# EUROPEAN PATENT APPLICATION

(11) **EP 3 827 800 A1**
(43) Date of publication of application: **02.06.2021**
(21) Application number: 19212155.6
(22) Date of filing: 28.11.2019
(51) Int. Cl.: A61G 13/12, A61B 8/00

(54) **A MECHANICAL SYSTEM WITH AN ADJUSTABLE MECHANICAL SUPPORT SYSTEM FOR CARRYING A DEVICE CONFIGURED TO CONTACT A HUMAN BODY OR AN ANIMAL BODY, A BODY POSITIONING SYSTEM AND AN ADJUSTABLE FRAME FOR HOLDING AN AUXILIARY TOOL**

(71) Applicant: Aison Technologies AG, 8406 Winterthur (CH)
(72) Inventor: The designation of the inventor has not yet been filed
(74) Representative: Frenkel, Matthias Alexander

(57) **Abstract**

The present disclosure relates to a mechanical system which comprises an adjustable mechanical support system (100) configured to carry a device (122) intended to be in contact with a human body or an animal body, wherein the adjustable mechanical support system (100) comprises one or more guiding elements (102, 104, 106), wherein each guiding element (102, 104, 106) extends in a respective main direction and having an adjustable length dimension in the respective main direction. The adjustable mechanical support system (100) is configured to slidably connect the device (122) with the one or more guiding elements (102, 104, 106).

## Description

The present invention generally relates to mechanical systems and more particularly to mechanical medical systems, in particular to medical imaging systems, to mechanical cosmetic systems and to mechanical care systems. More specifically, the present invention relates to a mechanical system configured to carry a device configured to contact a human body or an animal body. The device may be a medical device such as a medical diagnostic device, for example an ultrasonic device, a care device and/or a cosmetic device.

Physical exercise can lead to injuries, in particular of muscles, ligaments, tendons and the like. Such soft-tissue injuries as well as soft-tissue diseases are relevant in the fields of orthopaedics, rheumatology and neurology. It is desirable to detect and observe such injuries and diseases in a consistent, accurate and reproducible way. A common non-invasive imaging technique, for example, is ultrasound imaging. Soft-tissue measurements by current ultrasound imaging systems, such as for example freehand ultrasound systems, often suffer from the problem that the measurement results depend on the operator's handling of the ultrasound device. For example, the resulting medical images are dependent on the exerted pressure and the angle the operator uses with respect to the body surface. If, for example, the hand is rotated a bit with respect to the previous day, the whole anatomy looks different. Furthermore, technical parameters like the defined focal point, the used frequency, brightness and the contrast, the imaging mode and the ultrasound transducer choice also have a not negligible effect on the measurement result. Consequently, for having ultrasound data to be recorded in a consistent and desirable way, which again allows an easy comparison of the anatomical and physiological measurement data taken on consecutive days, it is decisive to remove the operator dependence.

For example, a known solution for removing the operator dependence uses a full-fledged robotic arm that can move freely and precisely on a human's body. However, solutions with full-fledged robotic arms are expensive, huge in size and there are concerns regarding the safety. Even though the robot may be programmed in such a way that it is absolutely safe, the system itself is not inherently safe because the motors used thereon have to be strong enough to overcome a certain lever, for example, for being able to flexibly reach other body parts. This gives rise to stronger regulations and the costs involved with proving that those regulations are put in place are high.

In addition, solutions based on a robotic arm are typically intended to be used by skilled staff which restricts the range of potential operators of the device. Furthermore, since their implementations are relatively large, a dedicated room in a hospital, surgery, or medical office is needed and an easy transportability is thereby not possible.

Other known solutions for removing the operator dependence use a mechanical system with fully active components having fewer axes, therefore having the potential to be safe by design because the motors which are needed to move the system may be less powerful. However, those systems are fixed in size and therefore are not modular. Hence, the usability for small body parts is restricted and inaccuracies or mistakes during the measurement can occur more easily. Furthermore, such a device is not easily disassembled.

Furthermore, also a replicable position of a subject's body with respect to the mechanical system is decisive for having consistent and reproducible measurement results. There are currently available body position control systems that are used in conjunction with magnetic resonance imaging (MRI). These systems involving MRI may scare a lay person and may make them feel constricted. There are also known solutions intended to be used with ultrasound, which involve the skin getting into touch with water. The solution involving water has the downside that the box in which the hand is immersed needs to be thoroughly cleaned. Even though this is a very well-functioning low-cost implementation from a technical point of view, issues can arise when the hand to be immersed in water has an open wound or a patient an infectious disease.

In addition, in case of ultrasound measurements, for example, it is known to use accessories and devices that make the measurement easier and that are adapted to minimize the amount of ultrasound gel to be used. Examples for such accessories are gel pads and reusable gel cushions. These accessories are dedicated to the scan of near-field areas and of irregular surfaces of the body, like hands. Also known are frame supported membranes that are placed between the skin and the ultrasound probe to keep firm tissues during the scan. Those systems can be found mainly on ultrasound devices for breast scan and are typically connected to a robotic arm. Common frames for such accessories and devices are configured to be attachable to a mechanical system that also contains a body position control system. Further, they have a fixed size.

Accordingly, there is a need for a mechanical system that is small in size and nevertheless usable for body parts of every size, transportable and that produces reliable and reproducible measurement results.

According to a first aspect, a mechanical system is provided which comprises an adjustable mechanical support system configured to carry a device configured to contact a human body or an animal body, wherein the adjustable mechanical support system comprises one or more guiding elements, and wherein each guiding element extends in a respective main direction and has an adjustable length dimension in the respective main direction. The adjustable mechanical support system is configured to slidably connect the device with the one or more guiding elements.

The mechanical system may be a purely mechanical system but is not limited to a purely mechanical system. For example, the mechanical system may also comprise electric elements, electronic elements, hydraulic elements etc.

The adjustable mechanical support system may be adjustable to a size of a subject's body part, thereby allowing the measurement of smaller and larger body parts and of body parts of children and of adults. Thus, the adjustable mechanical support system is modular in size. At least a part of the adjustability is achieved by means of one or more guiding elements of the adjustable mechanical support system that have an adjustable length dimension in the main direction. The extension in length and reduction in length of the guiding rail may be implemented as being continuous or in fixed steps. Furthermore, the adjustment may be performed manually, i.e. purely mechanical, or automatically by a motor, such as a stepper motor or a servo motor.

The adjustable mechanical support system may comprise, in addition to the adjustable guiding elements, further supporting elements, such as rods, that may be connected to the guiding elements and may thus form part of the mechanical support system.

The device may be any device which is intended to come into contact with a human body or an animal body, i.e. that, when being in operation, is in contact with a human body or an animal body. The device may be a medical device such as a medical treatment device and/or a medical diagnostic device. The medical diagnostic device may be a medical imaging device such as an ultrasound transducer (e.g. linear, curvilinear, or other) or a measurement system consisting of a combined thermographic and ultrasound unit. The device may be a care device used, for example, in connection with the care of older and/or people in need. The device may also be a cosmetic device such as a razor, for example. It also may be any other electronic or mechanical system that could be steered and can be brought into contact with a human or animal body. The device may be held by a core system that can be slidably connected with the one or more guiding elements. The device may be also directly slidably connected with the one or more guiding rails.

The guiding elements may be made of any rigid material, single or composite, having a small expansion coefficient in the range of temperatures generally used in the specific intended application field. Typical examples for the guiding elements are metal (e.g. aluminium), carbon-based composite materials or plastic.

Slidably connected with the one or more guiding elements encompasses the possibility that the (direct or indirect) connection with the guiding element is implemented as a sliding connection. Slidably connected with the one or more guiding elements also encompasses the possibility that the device is (directly or indirectly) fixedly connected to a guiding element or another element, wherein the guiding element or the other element is (directly or indirectly) slidably connected with another guiding element.

Slidably connected may mean that the specific elements may slide along the main direction of the guiding element to which it is slidably connected. The sliding motion may be realized purely mechanically or automatically by a motor. If the sliding motion is not operated by motors but purely mechanical, the device can either freely be steered by hand (as predetermined by the sliding connection) or within a predetermined path and/or structure. In the latter case, the path may be set by additional guiding elements attached to a core sliding system enabling the sliding connection of the device with the one or more guiding elements, the additional guiding elements extending in an x- and y-direction, having sliding rails and shaped as a fork with only two arms or as half a rectangular function. If the motion is operated by motors, the device is moved along a path that is dictated by a software. Thus, the motion is managed by a controller piloted by a computer. The path is limited by the configuration of the mechanical support system, i.e. the allowed movement along the guiding rails. A position tracking system may measure where the device such as the ultrasound transducer is precisely located with respect to a subject's body and within the structure of the mechanical support system. A tracking system can be implemented with a set of mirrors to define the precise location, or by revisiting a particular place on the body at the beginning of each measurement. Such a place is defined by where the underlying anatomy does not change with the exerted pressure on the skin, nor a slight rotation of the body, and which is furthermore unique. This could for example be an assembly of blood vessels or a specific feature of each bone. It may be also possible to provide a hybrid system, i.e. a motor-driven mechanical system, where, for example, an operator of the device may change the position of the device when the motor or motors stop. When the operator has then set the device to a new position, the operator may click a button and the mechanical support system may move again on its own. A new position may change the angle or the overall position of the device.

In an implementation of the first aspect, the mechanical support system comprises two or more guiding elements, wherein at least two of the two or more guiding elements extend in different main directions and are slidably and detachably connected to each other, wherein, optionally, the different main directions are perpendicular to each other. Thus, according to this implementation, the size of the mechanical support system is variable in at least two different main directions, which may be perpendicular to each other. Furthermore, the guiding elements are detachably connected, so that the system can be disassembled and easily transported.

In a variant of this implementation, the adjustable mechanical support system may comprise three or more guiding elements, wherein at least two guiding elements of the three or more guiding elements are arranged with a distance from each other and extend in parallel to each other, and wherein the two guiding elements of the three or more guiding elements are adjusted to have the same length dimension in the respective main direction. A third guiding element of the three or more guiding elements extends in a respective main direction being perpendicular to the respective main directions of the two guiding elements of the three or more guiding elements and has a length dimension corresponding to the distance between the two guiding elements of the three or more guiding elements, wherein the third guiding element is slidably connected with each of the two guiding elements. Also in this variant, slidably connected may be directly or indirectly slidably connected, so that the third guiding element may be also connected with the two guiding elements via other elements, and that the sliding connection may be implemented at the connection between the third guiding elements and the other elements and/or at the connection between the elements and the two guiding elements. Furthermore, according to this variant, the two guiding elements may have the function of supports for supporting the rest of the mechanical support system.

In this variant, for example, the two guiding elements may be connected with the third guiding element via a differently configured connecting element, but they may be also connected with the third guiding element via other guiding elements. The connecting element may be also configured to be variable in a length dimension, or the connecting element may be configured to be not variable in the length dimension. The connecting element may be configured as a pneumatic system, a double screw system, or may comprise a scissor lift mechanism, for example. The length of the connecting element may be adjusted mechanically or automatically through a stepper motor or a servo motor.

In an embodiment of this variant, the at least two guiding elements of the three or more guiding elements which are arranged with a distance from each other and extend in parallel to each other may be connected to each other by a stabilizing element extending in a direction perpendicular to a main direction of the at least two guiding elements. The stabilizing element may be a stabilizing additional guiding element or a stabilizing plate, for example. The stabilizing plate may be a telescopic plate with preset holes connecting the at least two guiding elements with each other or a flat surface with telescopic elements for adjusting. The stabilizing additional guiding element may be extendable and may be connected to the longitudinal ends of the two guiding elements. The stabilizing element may extend in the same plane or in a parallel plane as defined by the at least two guiding elements. Connecting the two guiding elements by a stabilizing element allows keeping the distance between the two guiding elements constant over the whole length of the two guiding elements so that they are located in parallel to each other.

According to a further implementation, the one or more guiding elements may each comprise two or more guiding sections that are configured to be movable into each other, wherein, optionally, the two or more guiding sections have a generally parallelepipedal shape or a cylindrical shape. By moving the guiding sections into each other, the length dimension of the guiding element can be varied. Thus, according to the further implementation, the one or more guiding elements may be configured as telescopic elements. The two or more guiding sections having a generally parallelepipedal shape may be configured as guiding rails. The guiding elements may have flat surfaces, but they also may have a profiled surface.

In an example of the further implementation, a guiding section of the two or more guiding sections comprises recesses cooperating with correspondingly shaped protrusions of another guiding section so as to be movable into each other. In this example, the outer dimensions of the guiding element are the same over the whole length of the guiding element, i.e. the outer dimensions are the same in a region where the guiding sections are moved into each other as in a region where the guiding sections do not overlap.

In an embodiment, the mechanical system comprises a sliding system for slidably connecting at least two guiding elements with each other and/or the device with the one or more guiding elements, wherein the sliding system comprises a sliding sleeve having an outer cross-sectional shape corresponding to the outer cross-sectional shape of the respective guiding element and has a gear element cooperating with a corresponding gearing formed on an outer surface side of the guiding element. When the guiding element has a rectangular cross-section, the gearing may be formed on at least one side surface. Generally, the gearing may be formed on at least one quarter of the outer surface of the guiding element. Thus, the slidable connection is provided by the sliding system. In this embodiment, a motion of the sliding sleeve is realized by the gear element rolling over the gearing, in particular it may be realized by a motor which is connected to the gear element thereby enabling a rolling of the gear element over the gearing. The gear element may be cylinder-shaped having the same width as the corresponding outer surface side of the guiding element. The motor for the movement of the sliding sleeve may be hosted by the sliding system.

In this embodiment, the sliding system may comprise bearing elements for slidably bearing the sliding sleeve against the respective guiding element. The bearing elements may be cylindrical-shaped, for example in the case of guiding elements with flat surfaces. They may be also constructed with wheels, for example in case of guiding rails with profiled surfaces. The may be placed or formed on all side surfaces of the guiding element, except the side surfaces where the gearing is provided.

According to a second aspect, the mechanical system comprises a body positioning system which is configured to be mounted to the adjustable mechanical support system and to positionally fix a body part of a subject's body with respect to the adjustable mechanical support system, wherein the body positioning system is configured to be adjustable to a size of a body part of a subject such as a patient. The body positioning system ensures that the body part to be measured may be put into a position that can be taken in the same way repeatedly. In particular, since it may be mounted to the adjustable mechanical support system, the relative position between the body positioning system and the mechanical support system may be fixed. Since the body positioning system ensures that the body part to be examined and/or treated and/or cared may be reproducibly placed at the same position, also the relative position between the body part and the mechanical support system carrying the device may be fixed.

In an implementation of the second aspect, the body positioning system may comprise a plate which is configured to be mounted to the adjustable mechanical support system and a protruding element which is provided on a first surface side of the plate so as to form a funnel for positionally fixing the body part, the funnel having a first opening and a second opening being smaller than the first opening. The plate with the funnel may be used both for measuring body parts of the upper limbs and the lower limbs. Depending on the body part to be measured, the plate may be placed parallel to a (ground) surface on which the mechanical system is placed, or it may be inclined with respect to the (ground) surface. The plate may comprise one or two connectors by means of which the plate may be hooked into the mechanical support system.

The body positioning system may comprise a block movably provided on the first surface side of the plate and having a shape corresponding to a negative shape of a palm of a hand, a hand-separating member movably provided on the first surface side of the plate, or a slider movably provided on the first surface side of the plate, wherein the slider has two slider elements which are arranged opposite to each other and wherein the slider is configured to vary a distance between the two slider elements. The block, the hand-separating member and the slider are all movable for enabling an adjusting to the size of the body part to be positioned by the block, the hand-separating member and the slider. The hand-negative shaped block may be for positioning a hand when measuring another body part of the upper limb such as the arm or elbow. The hand-separating member may be for positioning and measuring a hand. The slider may be used for positioning a foot when measuring a body part of the lower limb. The hand-separating member may be also used as a toe-separating member, i.e. for measuring the foot and optionally also positioning a foot. The block, the hand-separating member and the slider may be provided on different plates. In the alternative, the block, the hand-separating member and the slider may be interchangeably provided on a same plate. Or, in other words, depending on the application field, the same plate may comprise the block, the hand-separating member or the slider.

In a variant of the second aspect, the protruding element may be provided at a lower region of the plate and the block, the hand-separating member or the slider may be provided at an upper region of the plate opposite to the lower region, wherein the block and the hand-separating member are configured and arranged so as to be movable towards the protruding element, and the slider is configured and arranged so that a distance between the slider elements in a direction perpendicular to or at least inclined with respect to a length direction of the funnel formed by the protruding element is variable. In this variant, the funnel together with one of the block, the hand-separating member and the slider is provided on the same plate. Also here, the block, the hand-separating member and the slider may be interchangeably provided on a same plate. The funnel may be for positioning the heel or the hand wrist.

In a further variant of the second aspect, a box may be provided on a second surface side of the plate, wherein the box has a generally parallelepipedal shape and at least one open side. For using the second surface side, the plate may be flipped. The box may be used for a posterior measure of body parts of the lower limbs, where the patient lies in a prone position with outstretched legs, by sliding the foot into the box with the toes first. The box may have soft and round edges so as to provide further comfort to the person.

The adjustable mechanical support system may comprise a supporting block attached to a longitudinal end of a guiding element, wherein the supporting block comprises a slot for receiving the plate of the body positioning system. In that way, the plate may be positionally fixed with respect to the mechanical support system. The supporting block may be attached to the guiding element by generic mechanical connections like screws, tabs or a mechanical latching mechanism. Furthermore, the supporting block may comprise a plurality of slots, which are differently orientated.

According to a third aspect, the mechanical system comprises an adjustable frame for carrying an auxiliary tool having an adjustable length dimension in at least one direction. The adjustable frame is configured to be positionally fixed with respect to the adjustable mechanical support system. The auxiliary tool may be an auxiliary medical tool, an auxiliary cosmetic tool and/or an auxiliary care tool. In the case of ultrasound measuring, for example, the auxiliary tool may be an auxiliary diagnostic tool, for example an acoustic coupling element such as a waterbag or a gel pad which may provide a flat surface for the ultrasound transducer thereby allowing for a fixed sonic waves exposure angle. Due to the adjustable length dimension, it may be adapted to the size of the body part to be measured, treated and/or cared.

The adjustable frame may comprise a rectangular frame element and two or more legs supporting the rectangular frame element, wherein the rectangular frame element is configured to carry the auxiliary tool.

The adjustable frame may comprise one or more telescopic and/or foldable elements. For example, the two or more legs supporting the rectangular frame element may be telescopic and/or foldable. In case of more than one telescopic element, the telescopic elements may be detachably connected to each other to form the adjustable frame.

Further details, advantages and aspects of the present disclosure will become apparent from the following embodiments taken in conjunction with the drawings, wherein:
- Figs. 1a-1c: schematically show perspective views of an adjustable mechanical support system of a mechanical system according to an embodiment of the present disclosure, wherein Fig. 1b shows the mechanical support system of Fig. 1a in an expanded state and Fig. 1c shows the mechanical support system of Figs. 1a and 1b in an unmounted state;
- Figs. 1d-1f: schematically show the mounting of a core system carrying a medical diagnostic device to a sliding system according to a first implementation, wherein Fig. 1d illustrates a front view, and Figs. 1e and If illustrate side views;
- Figs. 1g-1h: schematically show a second implementation of the mounting of a core system to a sliding system, wherein Fig. 1g illustrates a front view and Fig. 1h illustrates a side view;
- Figs. 2a-2c: schematically show perspective views of three embodiments of a guiding element of the mechanical support system according to the present disclosure;
- Fig. 2d: schematically shows a guiding element according to an embodiment of Fig. 2c, wherein a gear rack is formed on an outer surface of the guiding element;
- Fig. 2e: schematically shows, in a cross-sectional view, a guiding element with a sliding system according to an embodiment of the present disclosure;
- Figs. 3a-3c: schematically show, in a perspective view (Fig. 3a) and in front views (Figs. 3b and 3c), embodiments of a body positioning system according to the present disclosure to be used with body parts of the upper limb;
- Figs. 4a-4d: schematically show an embodiment of a body positioning system according to the present disclosure to be used with body parts of the lower limb, wherein Fig. 4a shows a front view of the embodiment, Fig. 4b shows a perspective front view of the embodiment, Fig. 4c shows a side view of the embodiment and Fig. 4d shows a perspective side view of the embodiment;
- Figs. 5a-5d: schematically show embodiments of an adjustable frame for holding an auxiliary device;
- Figs. 6a-6c: schematically show an adjustable frame as illustrated in Fig. 5 mounted to a mechanical support system;
- Fig. 7: schematically illustrates an embodiment of a mechanical system with a mechanical support system, a body positioning system and an adjustable frame; and
- Figs. 8a-8b: schematically show an embodiment of a supporting block connected to a mechanical support system for positionally fixing a body positioning system.

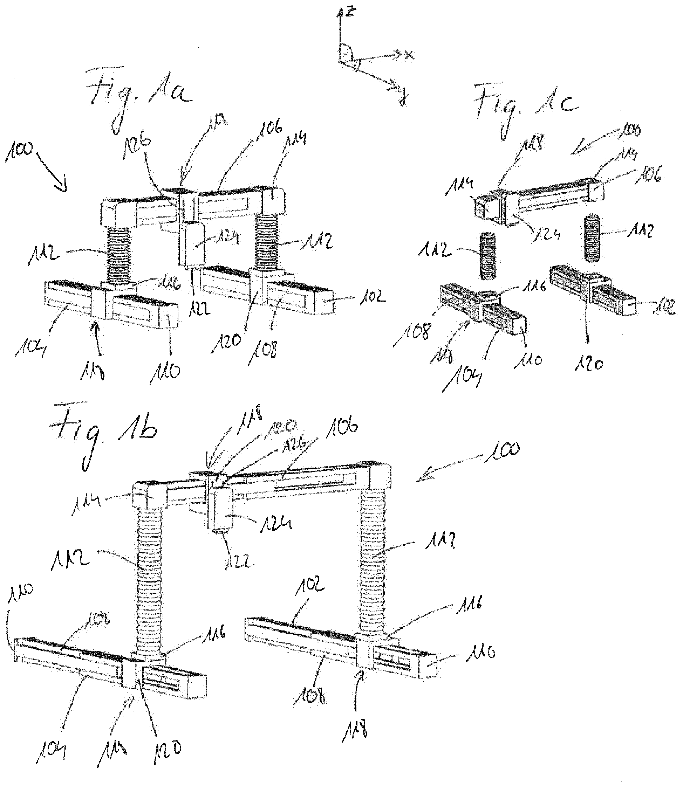

Figs. 1a to 1c show an embodiment of an adjustable mechanical support system 100. The adjustable mechanical support system comprises three guiding elements 102, 104, 106, wherein first and second guiding elements 102, 104 of the three guiding elements are arranged parallel to each other along a first direction and a third guiding element 106 is arranged along a second direction being perpendicular to the first direction. In the present embodiment, the respective first, second and third guiding elements 102, 104, 106 are embodied as guiding rails. Further, in the present embodiment, the guiding elements 102, 104, 106 have a generally parallelepipedal shape, in particular a rectangular parallelepipedal shape, more particularly a rectangular parallelepipedal shape with four rectangulars 108 and two squares 110 as side surfaces. Thus, a long side of a rectangular side surface 108 of a respective parallelepiped 102, 104, 106 extends in a direction which corresponds to a main direction of the respective parallelepiped 102, 104, 106, which is an extension direction of the respective parallelepiped 102, 104, 106. The above-described first and second directions correspond to the respective main directions of the respective parallelepiped 102, 104, 106.

Furthermore, the first direction corresponds to a y-direction, the second direction corresponds to an x-direction, and the third direction corresponds to a z-direction.

The third guiding rail 106 is connected at its two opposed end regions to the first and second guiding rails 102, 104 via two connecting elements 112. In the present embodiment, a connecting element 112 may look like a threaded cylinder from the outside, i.e. as a screw without a screw head. However, in the present embodiment, it is internally configured as a pneumatic system which allows changing the dimension of the mechanical support system in the z-direction. In alternative embodiments, the connection element 112 may be a double screw system that elongates the connecting element 112 to a desired length, or it may be of the same type as the guiding rails 102, 104 and 106. The connecting element 112 is connected to the first guiding rail 102 and the third guiding rail 106 via connectors, respectively, and the other threaded cylinder 112 is connected to the second 104 and third guiding rails 106 via connectors 114, 116, respectively. In the present embodiment, the connectors 114 at the third guiding rail 106 and the connectors 116 at the first and second guiding rails 102, 104 are embodied as an electromechanical system that allows the transmission of power and electrical signals from the guiding rails 102 and 104 to the guiding rail 106 through the connecting elements 112, respectively. The connectors 116 at the first and second guiding rails 102, 104 are part of a sliding system 118 slidably connecting the third guiding rail 106 to the first and second guiding rails 102, 104. In particular, the sliding system 118 comprises a sleeve 120 that at least partly surrounds the respective guiding rail 102, 104, 106 in a circumferential direction of the guiding rail 102, 104, 106. On an upper surface of the sleeve 120, an additional flat washer 116 having the function of the connector 116 is provided. The sleeves 120 are slidably mounted to the first and second guiding rails 102, 104, respectively, which will be described later.

The third guiding rail 106 carries a device 122 configured to contact a human body or an animal body. In the present example, the device is a medical diagnostic device 122, for example an ultrasonic transducer. In general, the device may be also any other electronic or mechanical system that could be steered and is provided to be in contact with a human or animal body. For that purpose, the third guiding rail 106 also comprises a sliding system 118 with a sleeve 120 to which a housing 124 of a core system carrying the medical diagnostic device 122 is mounted. As can be seen by comparing Figs. 1a and 1c with each other, the housing 124 with the medical diagnostic device 122 is mounted to the sleeve 120 so as to be movable in the z-direction. For example, the sleeve 120 may comprise rails and/or slots 126 cooperating with corresponding elements provided on the housing 124 (such as wheels, bushings, bolts and/or rods) for enabling a movement of the device 122 with respect to the sliding system 118 in the z-direction. Thus, by means of the sliding systems 118 as provided in the system of Figs. 1a to 1c, the medical diagnostic device 122 can be moved in the x-direction, the y-direction and the z-direction.

Furthermore, the housing 124 may be rotatable around a direction parallel to the y-axis and/or around a direction parallel to the x-axis.

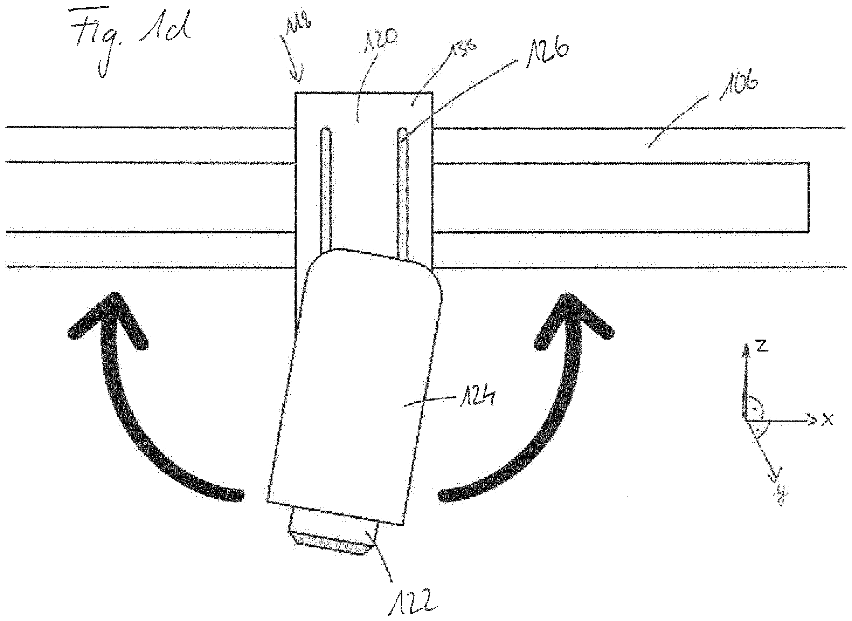

Possible implementations of the sleeve 120 of the sliding system 118 and the housing 124 for the medical diagnostic device 122 are illustrated in Figs. 1d to 1h.

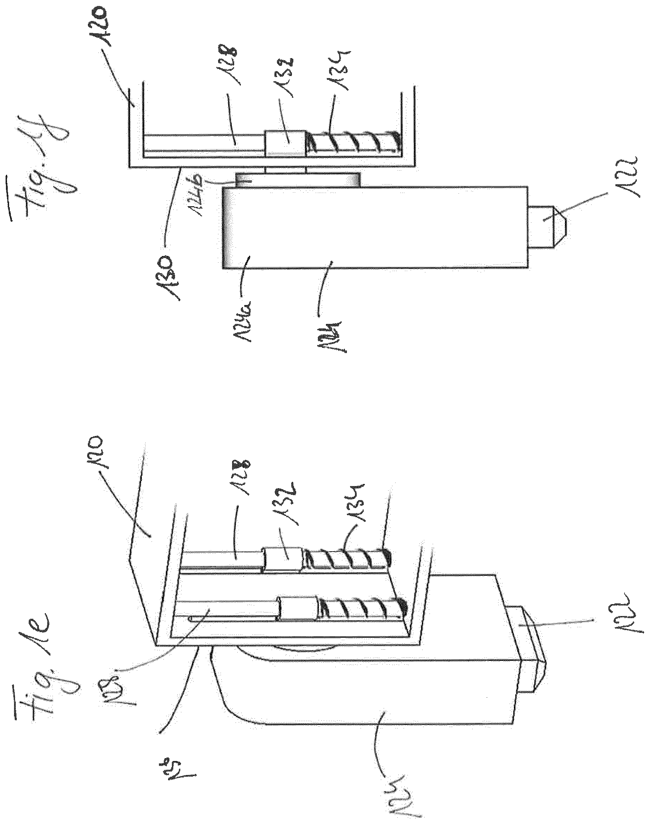

In a first implementation shown in Figs. 1d to 1f, the housing 124 is movable in a z-direction and rotatable around a direction parallel to the y-axis. As is seen in Figs. 1e and If, the movement in the z-direction is realized by means of a pair of cylindrical rods 128 provided inside the sleeve 120 behind a front side 130 of the sleeve 120. The front side 130 of the sleeve 120 further comprises two slots 126 which are positionally aligned with the pair of rods 128. The housing 124 is slidably connected to the sleeve 120 by means of a pair of bushings 132 each receiving a rod 128 of the pair of rods and being connected to the housing 124. In particular, in the present implementation, the bushings 132 extend through the slots 126 so as to be movable along the slots 126.

Figs. 1e and 1f further show that the housing 124 is preloaded with respect to the sleeve 120 and thus the mechanical support system. In particular, the bushing 132 surrounding the rod 128 rests on a spring 134 so as to be preloaded in a direction facing away from the intended position of a body part to be examined, treated and/or cared. In that way, the pressure exerted on a body part during the examination can be controlled.

The rotation of the housing 124 around a direction parallel to the y-axis is realized by the housing 124 comprising two housing parts being rotatable with respect to each other. Specifically, in the present implementation, the housing 124 comprises a front part 124a carrying the diagnostic device 122 and a rear part 124b connected to the sleeve 120. The front part 124a is connected to the rear part 124b so as to be rotatable in a direction parallel to the y-axis (cf. also Fig. 1d).

The sliding movement in the z-direction and the rotation around a direction parallel to the y-direction can be used to move the medical diagnostic device 122 to the intended examination position, wherein the springs 134 allow to control the pressure exerted on the body part in a z-direction. The rotation around a direction parallel to the y-direction can be, in addition, used for controlling the pressure exerted on the body part in this direction.

Further, in this example, the movement and thus the pressure control is fully passive, i.e. is fully non-motorised. In other examples, the movement may be fully active, i.e. motorised, or partly active and partly passive.

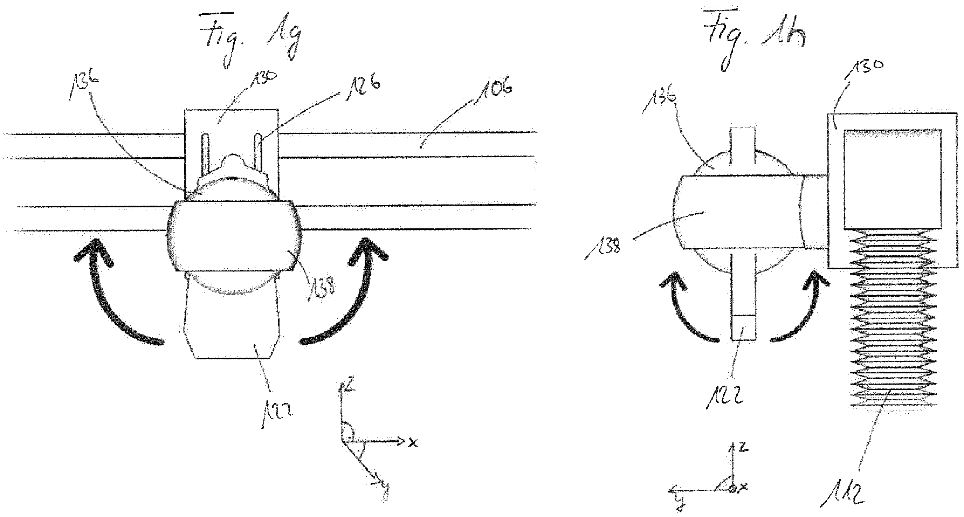

In a second implementation shown in Figs. 1g and 1h, the medical diagnostic device 122 is carried by a ball 136, which is rotatably mounted in a support 138. The support 138 is shaped to receive the ball 136 with the medical diagnostic device 122, in particular the support 138 is shaped with a correspondingly sized and shaped recess. The ball 136 in the support 138 is rotatable both around a direction parallel to the y-axis and around a direction parallel to the x-axis. The support 138 is mounted to the sleeve 120 of the sliding system 118 so as to be movable in a z-direction, which is only schematically illustrated. The support 138 may be mounted to the sleeve 120 in the same manner as the housing 124 is mounted to the sleeve 120 in the first implementation, i.e. by means of the rods 128, the slots 126 in the sleeve 120 and the bushings 132 surrounding the rods 128 and extending through the slots 126. In the alternative, the support 138 may be mounted in any other manner to the sleeve 120 which allows a movement in the z-direction.

The rotation in the direction parallel to the x-direction and in the direction parallel to the y-direction may be passively induced or actively steered by motors. In general, all movements, i.e. the movements in all directions, may be fully passive, fully active or may include a hybrid active and passive system, where one or more movements are steered by motors and the others not. Thus, since the above described movements of the housing and support with respect to the sleeve may also allow pressure control in the respective direction, the pressure feedback control in all space directions may be fully passive, fully active, or a hybrid of passive and active movements.

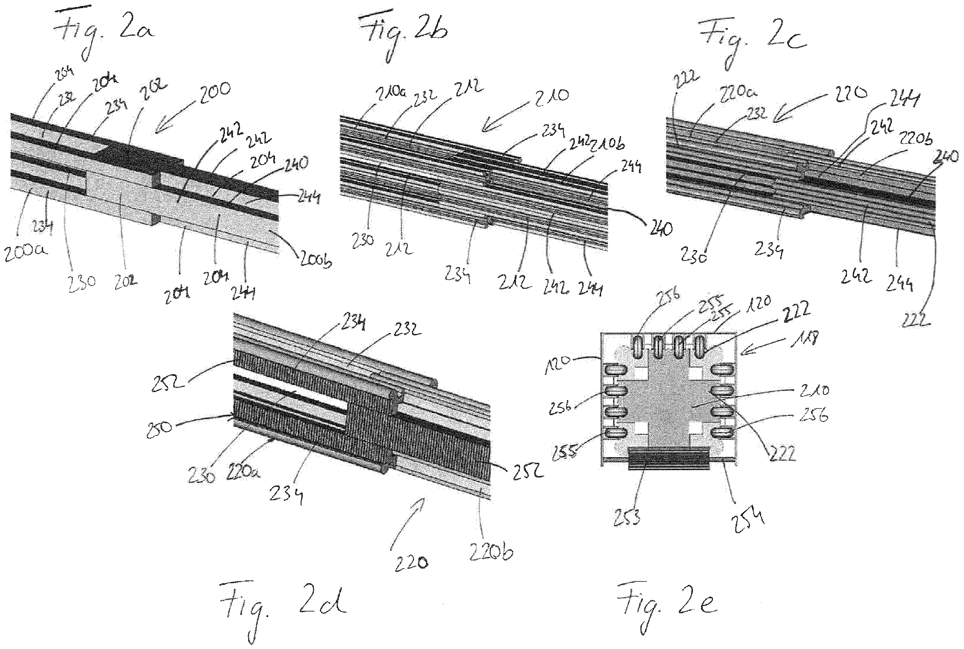

Fig. 1b shows the mechanical support system 100 in an expanded state compared to the compact, unexpanded state depicted in Fig. 1a. Specifically, the guiding rails 102, 104, 106 are configured to vary their length in their respective main direction, i.e. the guiding rails 102, 104, 106 have an adjustable length dimension in the respective main direction. More specifically, according to the present embodiment, the guiding rails 102, 104, 106 each comprise two guiding sections that are configured to change their relative positions in the main direction. The guiding sections are configured so as to be movable into each other in the main direction. For example, in an implementation, a first guiding section has recesses or cut-outs that cooperate with correspondingly shaped protrusions or solid regions of a second guiding section. Possible implementations of the guiding elements 102, 104, 106 will be discussed with reference to Figs. 2a to 2e.

Returning to the expanded state in Fig. 1b compared to the unexpanded state of Fig. 1a, due to the variable length of the guiding rails in their main direction and the variable length of the connecting elements 112, the mechanical support system can be adjusted in size in the y-direction, in the x-direction and in the z-direction. In this way, the size of the mechanical support system can be adjusted to the size of the body part to be examined, treated, and/or cared. The adjustment can be mechanical or driven by a motor. The movement of the guiding sections into each other may be implemented as a continuous movement or as a movement in fixed, predetermined steps.

As is illustrated in Fig. 1c, the adjustable mechanical support system 100 is configured to be unmounted for an easy storage and/or transportation, also by an end user. In particular, by disengaging the connections 114, 116 of the connecting elements 112 at the first to third guiding rails 102, 104, 106, i.e. by unscrewing the threaded cylinders 112 from the sliding systems 118 of the first and second guiding rails 102, 104 and from the end caps 114 of the third guiding rail 106, the mechanical support system 100 can be disassembled into the individual guiding elements 102, 104, 106 and connecting elements 112. Thus, the disassembled guiding elements in their compact dimension allow an easy transportation.

Figs. 2a to 2d show different examples of a guiding element 200, 210, 220 according to the present disclosure, wherein all examples show a guiding element 200, 210, 220 which is formed as a rectangular parallelepiped and comprises two guiding sections 200a, 200b, 210a, 210b, 220a, 220b which each are formed as a rectangular parallelepiped.

Fig. 2a shows a guiding element 200 with flat surfaces. In a compact, unexpanded state the guiding element 200 has four flat surfaces 202. In an expanded state, the guiding element 200 has several flat surfaces 204 or flat surface portions 204 (here: three surface portions 204 for each surface side) that are displaced to each other.

Fig. 2b shows a guiding element 210 with v-shaped cut-outs 212 formed on the side surfaces. In particular, the flat surface portions of Fig. 2a form v-shaped cut-out surface portions 212 in the example of Fig. 2b, so that several v-shaped cut-outs 212 (here: two for the guiding element in the expanded state and four for the guiding element in the unexpanded state) are provided for a surface side of the rectangular parallelepiped. If the guiding element has a gearing, at least one of the side surfaces has flat surface portions for receiving the gearing, as will be explained with reference to Fig. 2d. Fig. 2c depicts an example of a custom design of a guiding element 220, with u-shaped cut-outs 222 formed on the side surfaces.

Common to all three examples is that a first guiding section 200a, 210a, 220a has cut-outs and recesses at designated places, and that the corresponding second guiding section 200b, 210b, 220b has a solid construction at places corresponding to these designated places of the first guiding section 200a, 210a, 220a, and vice versa, so that the first 200a, 210a, 220a and second guiding sections 200b, 210b, 220b are movable into each other. In particular, in the present examples, the first guiding section 200a, 210a, 220a has a hollow construction 230 in an inside area of the guiding section. At surface areas surrounding the inside area, the first guiding section 200a, 210a, 220a has a recess 232 in a central region of a respective surface area and has a solid construction 234 at the edges of the respective surface area. Vice versa, the second guiding section 200b, 210b, 220b has a solid construction 240 in an inside area of the guiding section. At surface areas surrounding the inside area, the second guiding section 200b, 210b, 220b has a solid construction 242 in a central region of a respective surface area and has a hollow construction 244 at the edges of the respective surface area. Thus, a guiding section 200a, 210a, 220a (200b, 210b, 220b) comprises recesses 230, 232 (244) and protrusions 234 (240, 242) that are configured to cooperate with correspondingly shaped protrusions 240, 242 (234) and recesses 244 (230, 232) of another guiding section 200b, 210b, 220b (200a, 210a, 220a).

The guiding rails can be made of any rigid material (single or composite) having a small expansion coefficient in the intended temperature range.

The guiding rail 220 of Fig. 2d corresponds to the guiding rail 220 of Fig. 2c, but viewed from a different perspective. The upper surface of Fig. 2c is illustrated as a front surface in Fig. 2d. The front surface 250 of Fig. 2d is a flat surface formed by flat surface portions of the guiding sections (with the exception of a protrusion formed at the upper and lower edge coming from the v-shaped cut-outs of the adjacent side surfaces). On the flat front surface 250, a gearing 252 is formed, so that the guiding rail 220 forms a gear rack on that surface. More specifically, the first and second guiding sections 220a, 220b both have a gearing 252 on their front surface 250. The proper alignment of the gearing 252 on the two guiding sections 220a, 220b may be made by means of a mechanical system (not shown) fixing the two guiding sections 220a, 220b of the guiding element 220 at predetermined positions or through an electromechanical high precision positioning system (not shown). The gearing 252 is provided to cooperate with a gearing element 253 being part of the sliding system 118, as is described in the following with respect to Fig. 2e.

Fig. 2e is a cross-sectional view through a guiding element 220 surrounded by a sliding system 118. The guiding element may be the guiding element of Fig. 2d, wherein the lower surface side of Fig. 2e corresponds to the front surface with the gearing 252 of Fig. 2d. As can be seen in Fig. 2e, the gearing 252 cooperates with a gear element 253, in the present example a cylinder-shaped gear element 253, which is rotatably mounted on a shaft 254. The gear element 253 with the shaft 254 is part of the sliding system 118. The sliding system 118 further comprises the sliding sleeve 120. The sliding sleeve 120 at least partly surrounds the guiding rail 220. In the present example, the sliding sleeve 120 surrounds three side surfaces of the rectangular parallelepipedal guiding rail 220. At the fourth side surface, the gear element 253 with the shaft 254 is provided. The sliding system 118 comprises bearing elements 255, by means of which the sliding sleeve 120 slidably bears against the guiding element 220. In the present example, the bearing elements 255 comprise individual wheels 256 which are rotatably mounted to the sliding sleeve 120 and which engage into the u-shaped cut-outs 222 of the guiding rail 220.

In the present example, the bearing elements 255 are provided on all side surfaces of the guiding element 220 except for the side surface on which the gearing 252 is formed. In other implementations, the bearing elements 255 can be placed on fewer side surfaces, but it is preferred that elements (bearing elements 255 and/or gear elements 253) are not provided alone on a single side surface, but at least always on two opposed side surfaces of the guiding element. So, for example, bearing elements 255 are provided on two opposed side surfaces, or on one side surface bearing elements 255 are provided and on the other, opposed side surface a gear element 253 is provided. Further, in the present example, the guiding element 220 is a rectangular-shaped parallelepiped with the gearing formed on one side surface. The guiding element may also have a different shape, for example a cylindrical shape. In the implementation of the guiding elements with other shapes than the parallelepipedal shape, at least one quarter of the outer surface of the guiding element should be provided with a gearing.

The gear element 253 may be connected to a motor (not shown), such as a stepper motor or a servo motor. The motor may be hosted on the respective sliding system 118. The movement of the sliding system 118 is then managed by a controller piloted by a computer (not illustrated). Driving the gear element 253 by the motor results in a rolling of the gear element 253 over the gearing 252 of the gear rack and thus in a movement of the sliding sleeve 120 over the guiding element 220. Thus, the sliding system 118 allows the movement of any object connected to the sliding system 118 over the guiding rail 220. In the embodiment of Figs. 1a and 1b, the objects are other guiding rails and the core system with the housing 124. In particular, the sliding systems 118 on the first and second guiding rails 102, 104 allow a movement of the third guiding rail 106 and the core system in a y-direction, and the sliding system 118 on the third guiding rail 106 allows a movement of the core system in a x-direction as well as in a z-direction, and optionally in a direction around a direction parallel to the x-axis and/or a direction parallel to the y-axis.

The electric cables needed to power the mechanical support system 100 are provided in the guiding elements 102, 104, 106 and the electromechanical connecting elements 114, 116. The electric connections between guiding elements with different main directions may be realized by electric connectors inside the electromechanical connecting elements 114, 116 connecting the different guiding elements and/or axes with respect to each other.

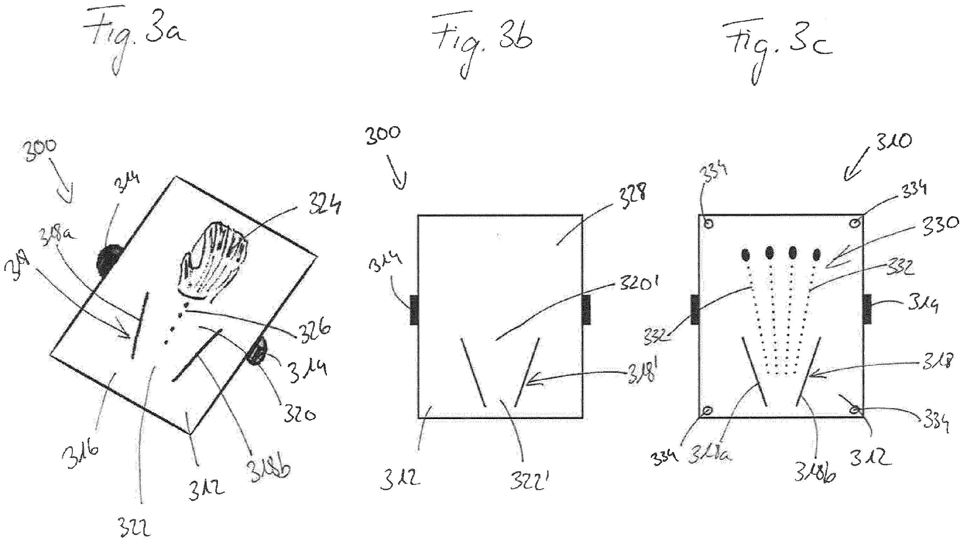

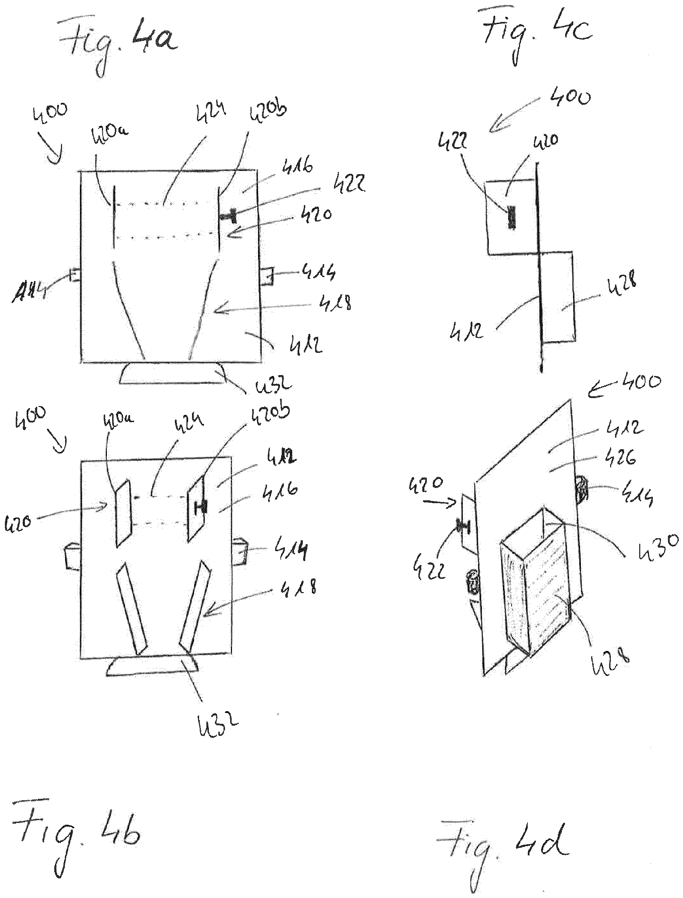

Figs. 3a to 3c show body positioning systems 300, 310 intended to be used when body parts of the upper limbs (arm, hand etc.) and foot are to be examined, treated and/or cared. Figs. 4a to 4d show a body positioning system 400 intended to be used when body parts of the lower limbs (leg, ankle, knee, etc.) are to be examined, treated and/or cared.

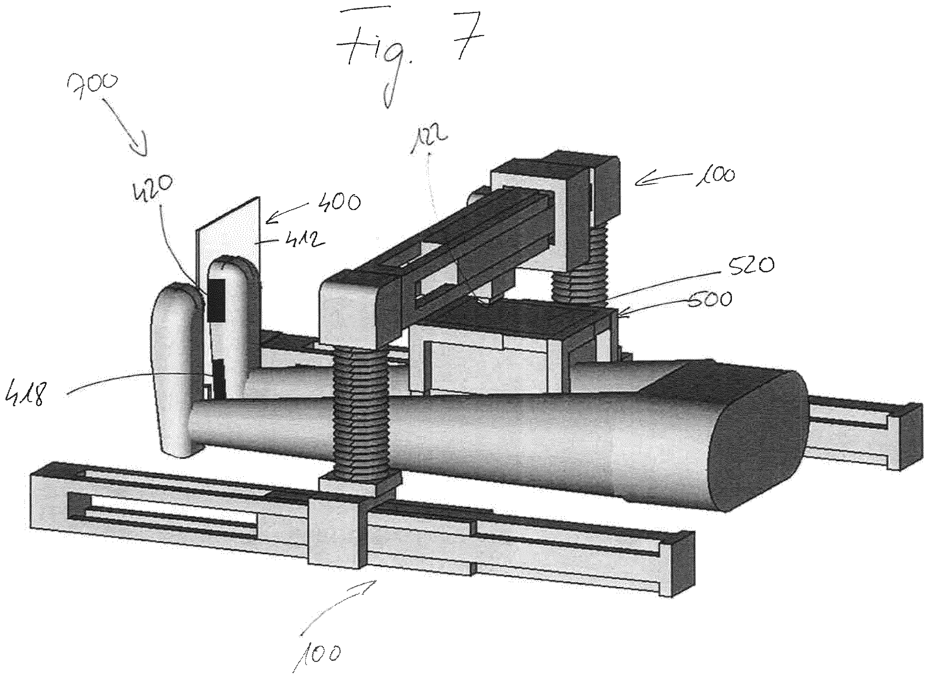

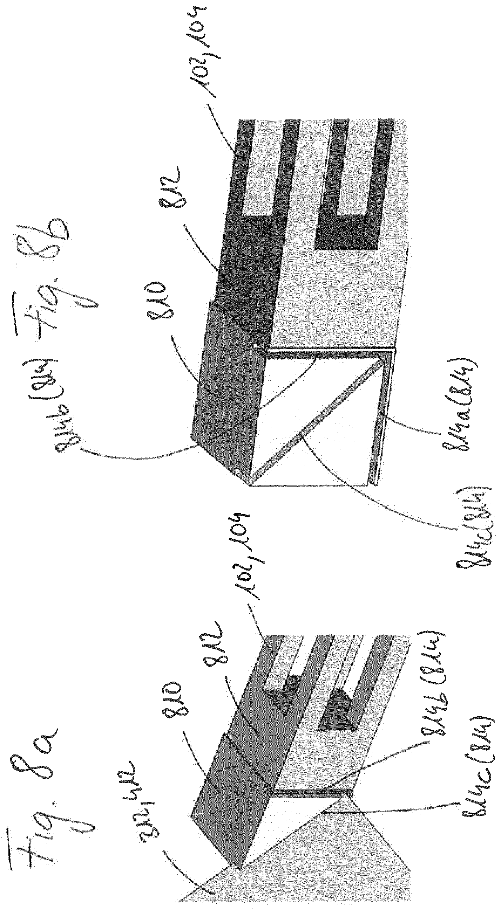

The body positioning systems 300, 310 of Figs. 3a to 3c, as well as that of Figs 4a to 4d, comprise a plate 312, 412 which may be made of plastic or any other material that can be easily cleaned and is robust enough. In addition, in case of an ultrasound transducer for the device, the material of the plate 312, 412 should minimize the reflection of ultrasound waves. The plate 312, 412 comprises a holding member 314, 414 on every side, for fixing the plate 312, 412 to the mechanical support system 100, in particular to the guiding elements. Thus, for example, the plate 312, 412 is fixed to a left hand side of the mechanical support system 100 if the left extremity of a patient is to be examined and to a right hand side of the mechanical support system 100 if the right extremity is to be examined. The holding member 314, 414 may be configured as tabs, hooks, straps etc. The holding members 314, 414 may also be located in other positions than shown here, for example there could be two holding members at one side that look like hooks or tabs (not illustrated in Figs. 3a to 4d). Furthermore, the plate 312, 412 is fixed to the mechanical support system 100 so that it can be inclined with respect to a ground surface of the system, as will be explained later with respect to Fig. 7 and Fig. 8.

On a front surface 316 of the plate 312, a funnel-shaped protrusion 318 is formed in a lower region, wherein the funnel-shaped protrusion 318 comprises two protruding elements 318a, 318b being arranged with respect to each other so as to form a funnel. Thus, at a lower end of the funnel, a first opening 322 is formed that is smaller than a second opening 320 formed at an upper end of the funnel. The two protruding elements 318a, 318b may be configured as panels protruding from the plate 312.

At an upper end of the plate 312 with respect to Fig. 3a, facing the second opening 320 of the funnel, a block 324 protruding from the front surface 316 is provided that has a shape corresponding to a negative shape of a palm of a hand. The block 324 has recesses that allow the individual fingers of a patient's hand to slide in and rest comfortably. As is indicated by a dotted line 326 in Fig. 3a, the block 324 is movable in a direction to the funnel. The movability may be realized by means of a slot or several slots receiving a correspondingly shaped element on the underside of the block 324, for example a pin or rail, for allowing the sliding of the block 324 along the slot or slots. A further possible implementation is a guiding rail formed on the plate 312 on which the block 324 is slidably mounted. Fig. 3a shows a rest position of the block 324. The block 324 is connected to the plate 312 by means of a biasing element, for example by means of a spring, enabling that the block 324 is automatically moved into the rest position when being displaced from the rest position and being released. The movement of the block 324 along the dotted line 326 allows an adjustability of the block 324 to fit to a patient's hand.

Fig. 3b shows the back surface 328 of the plate 312 of Fig. 3a. Thus, the back side of the plate only comprises a funnel-shaped protrusion 318' as has been explained with respect to Fig. 3a. In another implementation, the funnel-shaped protrusion 318' of Fig. 3b is formed on a separate plate 312 to that of Fig. 3a.

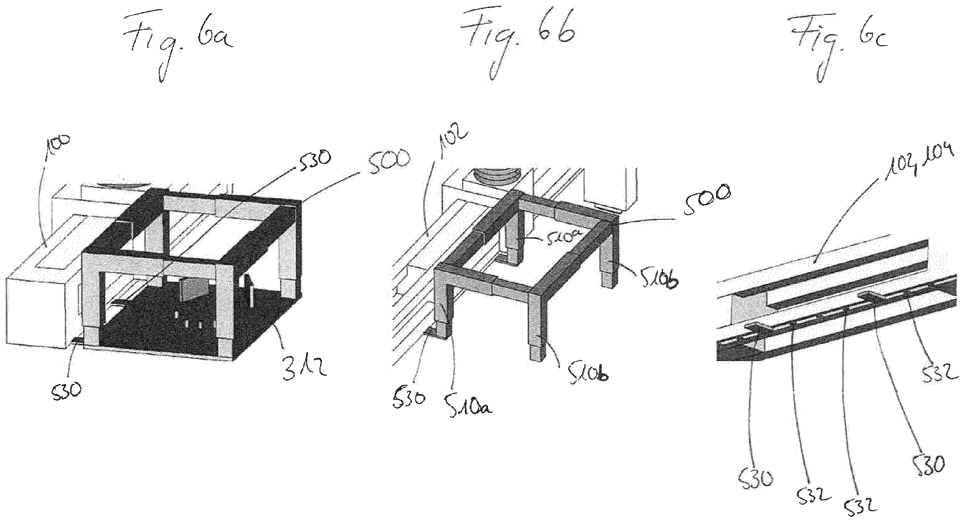

Fig. 3c shows another implementation of a plate 312 intended to be used when the foot or the hand are to be examined, treated and/or cared. Instead of the block 324 of Fig. 3a, a finger-separator 330 is provided at an upper end of the plate 312 facing the funnel-shaped protrusion 318. As is indicated by the dotted lines 332, the finger-separator 330 is also movably provided on the plate 312, in the same manner as has been described with respect to the block 324 of Fig. 3a. Furthermore, like the block 324 of Fig. 3a, the finger-separator 330 is biased in a rest position (cf. Fig. 3c) by means of a biasing element such as a spring. The finger separator 330 may be configured as four elements, for example as four bosses or knobs, protruding from the plate 312. The plate 312 further comprises four holes 334 at its corners for fixing an adjustable frame, as will be discussed with reference to Fig. 6a.

The two or three plates 312 depicted in Figs. 3a to 3c can be also combined into a single plate. In such an implementation with a single plate, the hand-shaped negative block 324 and the finger-separator 330 are detachably mounted to the plate 312, and depending on the kind of diagnostic application, the block 324, the finger-separator 330 or none of the both (cf. Fig. 3b) is provided on the plate 312.

The first plate 312 of Fig. 3a with the hand-shaped negative block 324 is provided when measuring the upper limb, and more particular, from the shoulder joint via an upper arm to the lower arm. Thus, the arm or a part thereof is the body part to be examined or measured, and a replicable positioning of the arm is achieved by means of a positioning of the hand. Specifically, when measuring that part of the upper limb, there are two positions necessary (dorsum position and palm position) in order for the medical diagnostic device such as the ultrasound to reach all soft-tissue locations. Both in the dorsum position and in the palm position, the arm is outstretched, i.e. it is extended at a 90° angle with respect to the torso. For both cases the body-position-control plate 312 of Fig. 3a can be used.

In both cases, LED lights (not illustrated) may guide the user to position the arm centrally within the mechanical support system. The status of the LED lights is determined from the measurements taken from at least two distance sensors (not illustrated). Alternatively, it would also be possible to introduce an additional body-position-control accessory that defines the position of the arm. This could be a board restricting the position of the arm from one or two sides. The board could be made of a plastic or other material that can be easily cleaned.

In the dorsum position, the object (here a person to be measured, treated, and/or cared, may be also an animal) lies in a supine position with an outstretched arm. In the following, the case of a person to be measured is described. Generally, the same applies for the other cases. The hand of the measurement object grabs the hand-negative shaped block 324 and slides to the bottom of the funnel-shaped protrusion 318, which is located on the plate 312. The palm of the hand touches the plate 312.

In the palm position, the measurement object (person to be measured) lies in a prone position with an outstretched arm. The hand of the measurement object slides to the bottom of the funnel-shaped protrusion 318, which is located on the plate 312. The back of the hand (dorsum) touches the plate 312. The hand-negative shaped block 324 is left in the original position (zero or rest position).

The plate 312 of Fig. 3c is used when measuring the hand. To allow for a replicable hand position, the hand wrist slides in the funnel-shaped protrusion 318 at the lower end. At the upper end, the adjustable finger-separator 330 can be moved along the dotted lines 332, for example within guiding rails, into the optimal position.

The plate 312 of Fig. 3c may be also used when measuring the foot (body part of lower limbs). In that case, for having a replicable foot position, the finger-separator 330 has the function of a toe-separator. The heel then slides into the funnel-shaped protrusion 318, and the finger separator 330, i.e. the toe separator, can be adjusted to a position optimal for toes, for example by moving the toes' separator within guiding rails. For measuring the foot, it is also possible to leave out the toe separation.

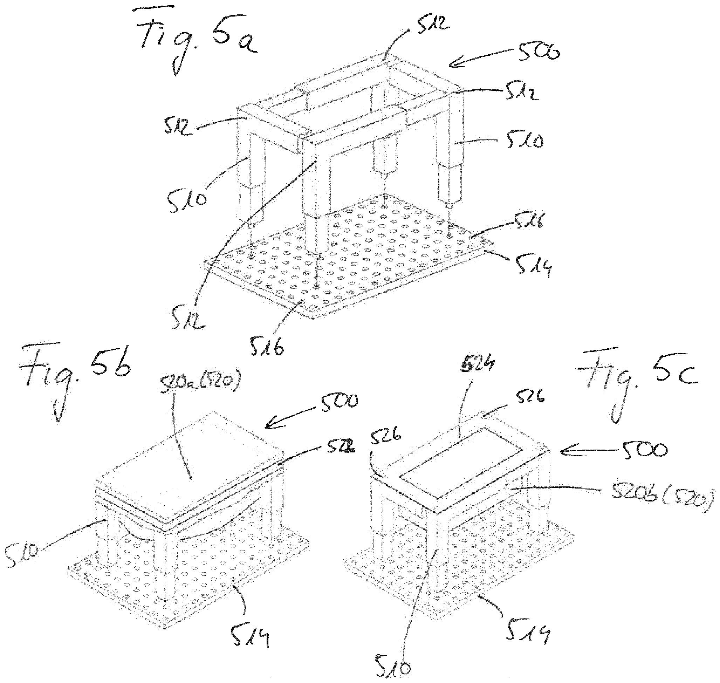

The plate 312 of Fig. 3c may have round recesses to connect with another measurement accessory, e.g. an adjustable frame 500, as will be explained with reference to Figs. 5a to 5c and 6a to 6c.

Figs. 4a and 4b show front views of a body-positioning system 400 intended to be used when body parts of the lower limbs (leg, ankle, knee etc.) are to be examined, treated and/or cared, Fig. 4c shows a side view and Fig. 4d shows a perspective view from the back side of the plate 412.

The front side 416 of the plate 412 comprises a funnel-shaped protrusion 418 in a lower area of the plate 412. The funnel-shaped protrusion 418 may be configured in the same manner as has been described with respect to the upper limb positioning system. In an upper area of the plate 412, opposite to the lower area, a slider 420 is provided that is movable in a horizontal direction of the plate 412, i.e. in a direction from the right to the left and vice versa with respect to Figs. 4a and 4b. In particular, the slider 420 comprises two slider elements 420a, 420b which are arranged opposite to each other and with a distance from each other, wherein the distance is variable by moving the two slider elements 420a, 420b towards and away from each other. More particularly, the slider 420 may be configured so that the slider elements 420a, 420b are symmetrically shifted away and towards each other, that means that a displacement of one slider element 420a, 420b towards or away from the other slider element 420b, 420a causes a corresponding displacement of the other slider element 420b, 429a towards or away from the one slider element 420a, 420b. For manually moving the slider elements 420a, 420b, at least one of the slider elements 420a, 420b comprises a handhold 422. Similar to the implementation of the plate 312 for the body parts of the upper limbs, the dotted lines 424 indicate the movability of the slider elements 420a, 420b, which may be realized by means of a slot or several slots receiving a correspondingly shaped element on the slider elements 420a, 420b, for example a pin or rail, for allowing the sliding of the slider elements 420a, 420b along the slot or slots. A further possible implementation is a guiding rail formed on the plate 412 on which the slider elements 420a, 420b are slidably mounted.

On the back side 426 of the plate 412, a box 428 with at least one open side 430 is provided, as is depicted in Figs. 4c and 4d. The box 428 is also provided in a lower area of the plate 412, opposite to the funnel-shaped protrusion 418 on the front side 416. The at least one open side 430 is formed at the side facing in a direction to the top (upper area), as is illustrated here, or to the bottom (lower area) of the plate with respect to Figs. 4c and 4d.

The body positioning system 400 of Figs. 4a to 4d comprises a support 432 on which the plate 412 is mounted. The plate 412 can be mounted at different angles to the support 432 to change the inclination of the plate 412 in a direction from left to right with respect to Fig. 4a, and vice versa, thereby for example allowing the leg to move from the anterior to the medial and lateral positions in a reproducible way. In an alternative implementation, the body positioning system 400 does not comprise a support, but the plate 412 is configured as having an inner circle and the rest of the plate being a rotating plate rotating around the inner circle.

In the following, it is described how the plate 412 of Figs. 4a to 4d can be used for measuring a body part of the lower limb (from the hip joint via the thigh to the lower leg).

When measuring the lower limb, there are four positions necessary in order for the medical diagnostic device such as the ultrasound transducer to reach all soft-tissue locations and get high-quality data.

As described with respect to the positioning for the upper limbs measurements, in all four positions, LED lights may guide the user to position both legs centrally within the mechanical support system. The status of the LED lights may be determined from the measurements taken from at least two distance sensors. The mechanical support system is extended to the point where the whole lower limb including the hip is placed inside the mechanical support system. Alternatively, it would also be possible to introduce an additional body-position-control accessory that defines the position of the leg. This could be one or two boards restricting the position of the leg from one or two sides. The board could be made of a plastic or other material that can be easily cleaned.

To create reproducible images of body parts of the lower limb, the foot needs to be fixed in such a way that its position is replicable. For the first three positions, i.e. the anterior, medial and lateral position, the same body position system is used, i.e. the front side 416 of the plate 412 (cf. Figs. 4a and 4b). For the fourth position, i.e. the posterior position, the plate is flipped to its back side 426 (cf. Fig. 4d).

In the anterior, medial and lateral positions, the measurement object (person to be measured) lies in a supine position with outstretched legs. The heel slides into the funnel-shaped protrusion 418 at the lower end of the plate 412. The slider 420 at the top of the funnel-shaped protrusion 418 restricts and fixes the position of the toes from two sides. Since the plate 412 can be rotated by a fixed angle in one (left) and the opposite (right) direction, the leg is allowed to move from the anterior to the medial and lateral positions in a replicable way.

In the posterior position, the measurement object (person to be measured) lies in a prone position with outstretched legs. A fixed-sized cushion may be placed below the knee in such a way that the lower leg is lifted and the toes do not touch the ground surface. The cushion may be a cushion that could be routinely used for a common ultrasound examination for limbs, for example any cushion that is easy to clean. The cushion to support the posterior position of the leg is as high as the largest foot. The cushion could also be an inflatable cushion with several segments to accommodate for different leg anatomies. The plate 412 is flipped to its other side, i.e. its back side 426 (cf. Fig. 4d). The plate 412 is inclined with respect to the ground surface by less than 90°. The foot, with the toes first, can slide into the box 428 at the lower end of the plate 412. The box 428 may but does not have to have protrusions for higher foot comfort.

The plate 412 of Figs. 4a to 4d may be a plate different from the plate 312 or plates illustrated in Figs. 3a to 3c. In the alternative, the different plates for the different parts of the body to be measured can be obtained by a single plate by changing the characteristic positioning elements (finger-separator, hand-negatively shaped block, slider), that can be mounted or unmounted depending on the specific part of the body to be measured.

For measuring an object's torso (and including the neck), no particular body positioning system is needed. LED lights may guide the user to position the torso centrally within the mechanical support system. The status of the LED lights is determined from the measurements taken from at least two distance sensors.

The mechanical system according to the present disclosure may further comprise an adjustable frame that is adjustable in size in order to adapt it to the specific designated application field and the body part to be examined, treated and/or cared. The adjustable frame is attachable to the mechanical support system 100. The adjustable frame may further be configured to carry common measurement equipment that is aimed to improve and/or facilitate the measurement. Such known measurement equipment may be any kind of acoustic coupling element commonly used in ultrasound examinations. They are characterized by a flat surface facing the ultrasound probe and a high elasticity and a sufficient size to adhere to the body.

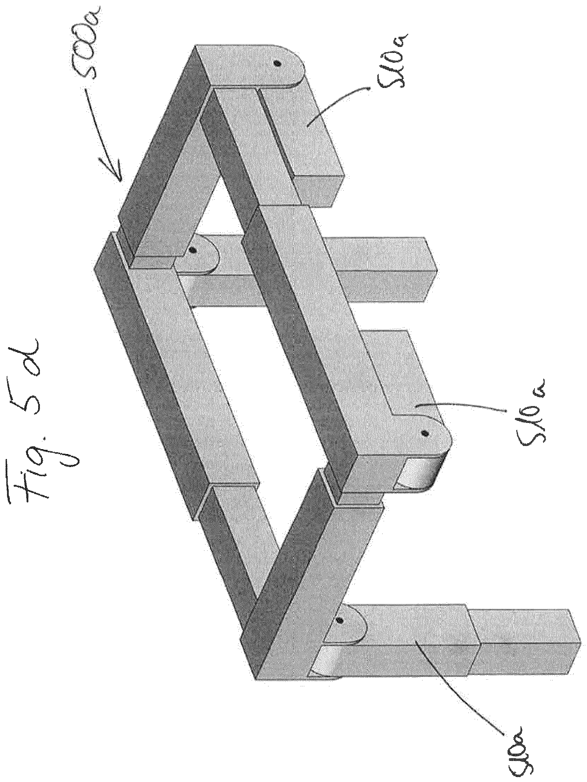

Fig. 5a shows an example implementation of an adjustable frame 500 with four legs 510 for having a good stability. In another implementation, the frame 500 may only comprise two legs. The frame 500 is adjustable in size since it is configured to be telescopic. In particular, it is variable in a height dimension, in a length dimension and in a width dimension. More particularly, the dimensions can be varied by fixed lengths, i.e. the telescopic elements can be elongated incrementally. The frame 500 may be assembled of individual telescopic elements. For example, in the depicted example of Fig. 5a, the frame 500 may be assembled of four telescopic L-shaped elements 512 mounted to each other. In another implementation shown in Fig. 5d, a frame 500a may comprise individual telescopic legs 510a that are also foldable.

In Fig. 5a, the adjustable frame 500 is connected to a mat 514 with holes 516. In particular, the holes 516 are arranged equidistantly in an array along columns and rows so as to be usable for different frame sizes. The mat 514 with holes 516 is part of a possible embodiment comprising the telescopic elements connecting the guiding rails 102, 104 extending in the y-direction with each other. The mat 514 is not telescopic itself in this embodiment. However, one may also envision the mat to be telescopic itself.

In Figs. 5b and 5c, two examples are shown for an acoustic coupling element 520 hosted by the frame 500 for keeping it at the same position during subsequent ultrasound measurements. In Fig. 5b, a common waterbag 520a is fixed with an elastic band 522 to the frame 500. The waterbag 520a can be closed or open at the top. If it is open, an additional proximity sensor has to be installed on the core system that makes sure the distance to the water is always kept fixed. Furthermore, the surface of the pertaining liquid, e.g. water, shall be flat. In Fig. 5c, a gel standoff pad 520b (reusable or not) is attached to a rigid frame 524 that is connected to the telescopic frame 500 by means of four elements 526 at the edges of the frame 500, such as screws or snap buttons. In another implementation, the gel standoff pad 520b may be also attached to a rigid surface that is translucent or practically translucent to ultrasound waves.

In Fig. 6a, the frame 500 is connected to a plate 312 of a body positioning system 300 by, for example, snapping the four legs into the four holes 334 seen in Fig. 3c. The plate 312 corresponds to the plate 312 of Fig. 3c for positioning and measuring a hand. The plate 312 of the body positioning system 300 is fixed to the frame 500, which in turn is fixed to the mechanical support system 100 by means of tabs 530.

In Fig. 6b, the adjustable frame 500 is connected to a first guiding element 102 extending in the y-direction. In particular, two legs 510a of the frame 500 are connected to the first guiding element 102 by means of tabs 530 connected to the legs 510a and the guiding rail 102. The other two legs 510b of the frame 500 may be connected to the bottom of the adjustable mechanical support system 100 (not illustrated in Fig. 6b). For example, they may be connected to a telescopic plate with preset holes connecting the first and second guiding elements 102, 104 both extending in the y-direction with each other, for example to a plate 514 as illustrated in Figs. 5a to 5c. The two legs 510a may be also connected to the telescopic plate or may only stand on the telescopic plate. In the alternative, they may be connected to a flat surface that can be adjusted by means of telescopic elements. In another implementation, the two legs 510b (as well as the legs 510a) may stand on a surface on which the whole mechanical system lays on, for example a mat or a treatment table.

Fig. 6c shows the guiding element 102 or 104 in an expanded state. Fig. 6c further shows how the hooks or tabs 530 are connected to a first guiding element 102, 104 extending in the y-direction. The guiding element 102,104 has small equidistant recesses 532 on the part of the guiding element 102, 104 facing the ground surface along its length dimension so as to be able to hook the tabs 530.

Fig. 7 shows an example of the mechanical system 700 according to the present disclosure, comprising an adjustable mechanical support system 100 with a core system carrying, for example, an ultrasound transducer for ultrasound examination, a body positioning system 400 and an adjustable frame 500. The adjustable mechanical support system 100 corresponds to the adjustable mechanical support system 100 illustrated in Figs. 1a and 1b. The body positioning system 400 corresponds to the lower limbs body positioning system 400 with the plate 412 comprising the funnel-shaped protrusion 418 and the slider 420 for positioning the foot, as is illustrated in Figs. 4a and 4b. As is seen, the heel is positionally fixed by the funnel-shaped protrusion 418, and the slider 420 restricts and fixes the position of the toes from the sides. Since the slider 420 is adjustable in size, the two slider elements 420a, 420b can be moved for coming into contact with the toes, thereby supporting the toes and fixing their position. The plate 412 is held by a supporting block with slots (further explained in Figs. 8a and 8b) on both sides, thereby positionally fixing the plate 412 with respect to the mechanical support system 100.

An embodiment of a supporting block 810 is shown in Figs. 8a and 8b. Fig. 8a shows the supporting block 810 with a plate 312, 412 of a body positioning system 300, 310, 400 fixed thereto, and Fig. 8b shows the supporting block 810 without any plate fixed thereto. The supporting block 810 is connected to the longitudinal ends, more specifically to end caps 812 at the longitudinal ends, of the guiding rail 102,104 by generic mechanical connections like screws, tabs or a mechanical latching mechanism. As can be seen in Fig. 8b, the supporting block 810 has three differently orientated slots 814 for orientating the plate 312, 412 in a different angle with respect to the mechanical support system 100. Specifically, in the present embodiment, a first slot 814a extends parallel to a longitudinal direction of the guiding element 102, 104, thereby allowing a horizontal orientation of the body positioning system 300, 310, 400 with respect to the mechanical support system 100. A second slot 814b extends perpendicular to the first slot 814a in a height direction of the guiding element 102, 104, thereby allowing a vertical orientation of the body positioning system 300, 310, 400 with respect to the mechanical support system 100. Finally, a third slot 814c extends in an inclined orientation with respect to the first and second slots 814a, 814b, thereby allowing an inclined orientation of the body positioning system 300, 310, 400 with respect to the mechanical support system 100. In the present configuration, the plate 312,412 is inserted into the slot 814c of the supporting block 810. In this way, the plate 312, 412 can be inserted at a fixed inclination angle. The plate 312,412 may be inserted into one the slots 814 of the supporting block 810 when measuring the upper or lower limb. When measuring the hand or foot, the plate 312, 412 is connected to the adjustable mechanical support system 100 through tabs, such as the holding members 314, 414 of Figs. 3 and 4. It is possible to use a second supporting block with slots to stabilize the plate 312, 412 when measuring the upper or lower limb. The second supporting block with slots is then not connected to a guiding element and has the matching mirrored slots of the supporting block 810 connected to the guiding element 102, 104.

The adjustable frame 500 carries an ultrasound coupling element 520. The leg extends through the frame 500, so that the upper part of the leg which is intended to be examined, treated and/or cared is located beneath the ultrasound coupling element 520. The ultrasound transducer 122 is located above the ultrasound coupling element 520.

The adjustable frame 500 is coupled to the adjacent guiding rail 102, 104 extending in the y-direction, for example in the manner as depicted in Fig. 6b and as has been described with respect to Fig. 6b. The size of the frame 500 is variable in the x-direction, in the y-direction and in the z-direction, thereby being optimally adapted to the dimensions of the upper part of the leg to be examined, treated and/or cared.

As has been described above, the mechanical support system 100 allows a movement of the core system and thus of the device 122 in the x-direction, the y-direction and the z-direction. Furthermore, depending on the configuration of the core system and the mounting to the slider, it may be also rotatable around a direction parallel to the y-axis and parallel to the x-axis. Thus, the device 122 can be moved in the three dimensional space. In that way, for example, several measurements from different positions in the three dimensional space can be taken, and the measurement results such as ultrasound images can then be stitched together to have measurements results from a full three dimensional volume. The three dimensional volume could be a full cylinder or alternatively also a hollow cylinder or a fraction of a full or hollow cylinder.

## Claims

1. A mechanical system (700), comprising:
an adjustable mechanical support system (100) configured to carry a device (122) configured to contact a human body or an animal body,
wherein the adjustable mechanical support system (100) comprises one or more guiding elements (102, 104, 106, 200, 210, 220), each guiding element (102, 104, 106) extending in a respective main direction and having an adjustable length dimension in the respective main direction, and
wherein the adjustable mechanical support system (100) is configured to slidably connect the device (122) with the one or more guiding elements (102, 104, 106).

2. The mechanical system (700) of claim 1, wherein
the mechanical support system (100) comprises two or more guiding elements (102, 104, 106), wherein at least two of the two or more guiding elements (102, 104, 106) extend in different main directions and are slidably and detachably connected to each other, wherein, optionally, the different main directions are perpendicular to each other.

3. The mechanical system (700) of claim 2, wherein
the adjustable mechanical support system (100) comprises three or more guiding elements (102, 104, 106), wherein at least two guiding elements (102, 104) of the three or more guiding elements are arranged with a distance from each other and extend in parallel to each other, the two guiding elements (102, 104) of the three or more guiding elements being adjusted to have the same length dimension in the respective main direction, and
wherein a third guiding element (106) of the three or more guiding elements extends in a respective main direction being perpendicular to the respective main directions of the two guiding elements (102, 104) of the three or more guiding elements and has a length dimension correlating with the distance between the two guiding elements (102, 104) of the three or more guiding elements, the third guiding element (106) being slidably connected to each of the two guiding elements (102, 104).

4. The mechanical system of claim 3, wherein
the at least two guiding elements (102, 104) of the three or more guiding elements which are arranged with a distance from each other and extend in parallel to each other are connected to each other by a stabilizing element extending in a direction perpendicular to a main direction of the at least two guiding elements (102, 104).

5. The mechanical system (700) of any of the preceding claims, wherein
the one or more guiding elements (200, 210, 220) each comprise two or more guiding sections (200a, 200b, 210a, 210b, 220a, 220b) that are configured to be movable into each other, wherein, optionally, the two or more guiding sections (200a, 200b, 210a, 210b, 220a, 220b) have a generally parallelepipedal shape or a cylindrical shape.

6. The mechanical system (700) of claim 5, wherein a guiding section (200a, 200b, 210a, 210b, 220a, 220b) of the two or more guiding sections comprises recesses (230, 232) cooperating with correspondingly shaped protrusions (240, 242) of another guiding section (200a, 200b, 210a, 210b, 220a, 220b) so as to be movable into each other.

7. The mechanical system (700) of any of the preceding claims, comprising
a sliding system (118) for slidably connecting at least two guiding elements (102, 104, 106) with each other and/or the device (122) with the one or more guiding elements (102, 104, 106),
the sliding system (118) comprising a sliding sleeve (120) having an outer cross-sectional shape corresponding to the outer cross-sectional shape of the respective guiding element (102, 104, 106) and, optionally, having a gear element (253) cooperating with a corresponding gearing (252) formed on an outer surface side of the guiding element (102, 104, 106).

8. The mechanical system (700) of claim 7, wherein
the sliding system (118) comprises bearing elements (255) for slidably bearing the sliding sleeve (120) against the respective guiding element (102, 104, 106).

9. The mechanical system (700) of any of the preceding claims, comprising
a body positioning system (300, 310, 400) configured to be mounted to the adjustable mechanical support system (100) and to positionally fix a body part of a subject's body with respect to the adjustable mechanical support system (100), wherein the body positioning system (300, 310, 400) is configured to be adjustable to a size of a subject's body part.

10. The mechanical system (700) of claim 9, wherein
the body positioning system (300, 310, 400) comprises a plate (312, 412) configured to be mounted to the adjustable mechanical support system (100) and a protruding element (318, 418) provided on a first surface side (316, 416) of the plate (312, 412) so as to form a funnel for positionally fixing the body part, the funnel having a first opening (320) and a second opening (322) being smaller than the first opening (320).

11. The mechanical system (700) of claim 9 or 10, comprising
a block (324) movably provided on the first surface side (316) of the plate and having a shape corresponding to a negative shape of a palm of a hand, a hand-separating member (330) movably provided on the first surface side (316) of the plate (312), or a slider (420) movably provided on the first surface side (416) of the plate (412), wherein the slider (420) has two slider elements (420a, 420b) which are arranged opposite to each other and wherein the slider (420) is configured to vary a distance between the two slider elements (420a, 420b).

12. The mechanical system (700) of claim 11, wherein the protruding element (318) is provided at a lower region of the plate (312, 412) and the block (324), the hand-separating member (330) or the slider (420) is provided at an upper region of the plate (312, 412) opposite to the lower region, and wherein the block (324) and the hand-separating member (330) are configured and arranged so as to be movable towards the protruding element (318), and the slider (420) is configured and arranged so that a distance between the slider elements (420a, 420b) in a direction perpendicular to or at least inclined with respect to a length direction of the funnel formed by the protruding element (318) is variable.

13. The mechanical system (700) of any of claims 9 to 12, comprising
a box (428) provided on a second surface side (426) of the plate (412), wherein the box (428) has a generally parallelepipedal shape and at least one open side (430).

14. The mechanical system (700) of any of claims 10 to 13, wherein
the adjustable mechanical support system (100) comprises a supporting block (810) attached to a longitudinal end of a guiding element (102, 104), wherein the supporting block (810) comprises a slot (814) for receiving the plate (312, 412) of the body positioning system (300, 310, 400).

15. The mechanical system (700) of any of the preceding claims, comprising
an adjustable frame (500) for carrying an auxiliary tool (520) having an adjustable length dimension in at least one direction and being configured to be positionally fixed with respect to the adjustable mechanical support system (100).
